# EUROPEAN PATENT APPLICATION

(11) **EP 4 759 819 A1**
(43) Date of publication of application: **17.06.2026**
(21) Application number: 23952781.5
(22) Date of filing: 27.09.2023
(51) Int. Cl.: C07F 9/30, C08K 5/3492, C08K 5/5313

(54) **AMMONIUM DIHYDROCARBYLPHOSPHINATE AND PREPARATION METHOD THEREFOR AND USE THEREOF**

(30) Priority: 18.09.2023 CN 202311206309
(71) Applicant: Jiangsu Liside New Material Co., Ltd., Taizhou, Jiangsu 225500 (CN); Ningbo Institute of Materials Technology & Engineering, Chinese Academy of Sciences, Ningbo, Zhejiang 315201 (CN)
(72) Inventor: LI, Jinzhong, Taizhou, Jiangsu 225500 (CN); YAO, Qiang, Ningbo, Zhejiang 315201 (CN); LU, Xiaodong, Ningbo, Zhejiang 315201 (CN); YANG, Jianwei, Taizhou, Jiangsu 225500 (CN)
(74) Representative: Santarelli
(86) International application number: PCT/CN2023/121955
(87) International publication number: WO 2025/060129

(57) **Abstract**

Provided are an ammonium dihydrocarbylphosphinate and a preparation method therefor and a use thereof. The ammonium dihydrocarbylphosphinate is obtained by alkylation reaction of an alkyl dihydrocarbylphosphinate and nitrogen on a non-tertiary amine structure in an organic nitrogen-containing compound. The ammonium dihydrocarbylphosphinate has a low melting point, is easy to liquefy, has good compatibility with resin, has a high compatibility speed and is easy to operate, so that the problems that solid amines have high melting points, are difficult to liquefy and are difficult to be compatible with resin systems are solved, and the problems of corrosiveness caused by dialkylphosphinic acid or a residue thereof, and the like are solved. The ammonium dihydrocarbylphosphinate, as a flame retardant, has high flame-retardant efficiency, and can effectively maintain the physical properties of a polymer material, especially the glass transition temperature. The ammonium dihydrocarbylphosphinate involves simple preparation, is free of a solvent and a catalyst, has high atom economy, and is suitable for large-scale production.

## Description

### Technical Field

The present invention belongs to the field of flame-retardant polymer materials, and relates to an ammonium dihydrocarbylphosphinate, preparation method therefor and use thereof.

### Background of the Invention

Polymer materials are widely used in various industries of the national economy. However, since most polymer materials are flammable, flame-retardant modification is often required to meet their flame-retardant requirements in order to reduce fire hazards caused by them. The simplest method of flame-retardant modification is to use flame retardants.

In recent years, phosphorus-based flame retardants have developed rapidly due to their characteristics such as high flame-retardant efficiency and little smoke and low toxicity of combustion products, and in particular, dialkylphosphinic acids and their salts have been applied.

CN110945047A discloses the product of the reaction between diethylphosphinic acid and epoxide, and its flame-retardant application in polyurethane foams. However, this product is a flame retardant with a monohydroxy functional group, which reduces the crosslinking degree of polyurethane and consumes expensive isocyanates. WO2021074684 discloses the use of diethylphosphinic acid or its product with epoxy resin for flame retardation of epoxy resins. However, the reaction between diethylphosphinic acid and epoxy resin reduces the crosslinkable functional groups of the epoxy resin, thereby requiring the use of low-exothermic epoxy resins with trifunctionality or higher. CN113929967B discloses the neutralization product of dialkylphosphinic acid and amine compounds and its application. Such products are solid, have poor compatibility with polymer materials, and suffer from the problem of poor uniform dispersion. EP3470444 discloses quaternary ammonium dihydrocarbylphosphinates for flame retardation of polyurethane, but quaternary ammonium salts lack reactive groups and are prone to precipitation and loss.

To sum up, due to their strong acidity, dialkylphosphinic acids can only be used after neutralization. However, neutralization often reduces the number of functional groups of the neutralizing agent, which is detrimental to the physical properties of flame-retardant materials. In addition, the existing solid ammonium dihydrocarbylphosphinates have problems such as poor compatibility with polymer materials, and quaternary ammonium dihydrocarbylphosphinates lack reactivity.

### Summary of the Invention

The following is a summary of the subject matter described in detail in this document. This summary is not intended to limit the scope of protection of the claims.

In view of the shortcomings of the prior art, the purpose of the present invention is to provide an ammonium dihydrocarbylphosphinate, preparation method therefor and use thereof.

To achieve this purpose, the present invention adopts the following technical solutions.

In an aspect, the present invention provides an ammonium dihydrocarbylphosphinate, which is obtained by an alkylation reaction between an alkyl dihydrocarbylphosphinate having the structural formula shown in Formula (I) and nitrogen on at least one non-tertiary amine structure in NH₃ or an organic nitrogen-containing compound, wherein the alkyl group participating in the alkylation reaction is derived from R₁ in the structural formula shown in Formula (I), the organic nitrogen-containing compound is selected from primary amines, secondary amines or imine compounds, and in the ammonium dihydrocarbylphosphinate, at least one nitrogen atom among the nitrogen atoms that have undergone the alkylation reaction has both an H atom and an R₁ group obtained through the alkylation reaction: 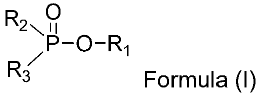
where R₁ is selected from the group consisting of C₁~C₁₈ straight-chain alkyl groups, C₁~C₁₈ branched-chain alkyl groups, and C₃~C₁₈ cycloalkyl groups; and
R₂ and R₃ are independently selected from the group consisting of C₁~C₁₈ straight-chain alkyl groups, C₁~C₁₈ branched-chain alkyl groups, C₃~C₁₈ cycloalkyl groups, and C₆∼C₂₄ aryl groups.

In an embodiment, R₁ is any one selected from the group consisting of methyl, ethyl, propyl, butyl, pentyl, hexyl, heptyl, octyl, nonyl and decyl.

In an embodiment, R₁ is any one selected from the group consisting of methyl, ethyl, propyl and butyl.

In an embodiment, R₁ is any one selected from the group consisting of methyl and ethyl.

In an embodiment, R₂ and R₃ are any one independently selected from the group consisting of methyl, ethyl, propyl, butyl, pentyl, hexyl, heptyl, octyl, nonyl, decyl and phenyl.

In an embodiment, R₂ and R₃ are any one independently selected from the group consisting of ethyl, propyl and butyl.

In an embodiment, R₂ and R₃ are ethyl.

The alkyl dihydrocarbylphosphinate having the structural formula shown in Formula (I) can be a pure alkyl dihydrocarbylphosphinate or a mixed alkyl dihydrocarbylphosphinate with different R₁, R₂ and R₃ substituents. For example, the alkyl dihydrocarbylphosphinate can be methyl diethylphosphinate, ethyl diethylphosphinate, or a mixture thereof.

In an embodiment, the alkyl dihydrocarbylphosphinate includes one or a combination of at least two selected from the group consisting of methyl dimethylphosphinate, methyl diethylphosphinate, methyl dipropylphosphinate, methyl ethylpropylphosphinate, methyl diphenylphosphinate, ethyl dimethylphosphinate, ethyl diethylphosphinate, ethyl dipropylphosphinate, ethyl ethylpropylphosphinate, propyl dimethylphosphinate, propyl diethylphosphinate, propyl dipropylphosphinate, propyl ethylpropylphosphinate, methyl methylphenylphosphinate and methyl diphenylphosphinate.

The organic nitrogen-containing compound is selected from the group consisting of primary amines, secondary amines and imines. In the present invention, a primary amine refers to a derivative of NH₃ molecule where one hydrogen atom is substituted by a (substituted) alkyl group; a secondary amine refers to a derivative of NH₃ molecule where two hydrogen atoms are substituted by (substituted) alkyl groups; and an imine, structurally equivalent to the product prepared by the condensation of ammonia or a primary amine with an aldehyde or ketone, has an amine structure as shown in Formula (II) below. 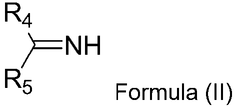 where R₄ and R₅ are independently selected from the group consisting of hydrogen, substituted or unsubstituted alkyl groups, and substituted or unsubstituted aryl groups.

In the structures of the primary amines, secondary amines and imines, a single molecule can contain multiple amino groups, and/or imino groups, and/or -C=NH groups simultaneously. For example, a primary amine can contain two amino groups, or one amino group and one imino group, or one amino group and one -C=NH group. When the structure of a primary amine, secondary amine or imine contains multiple amino groups, imino groups and/or -C=NH groups, the ammonium dihydrocarbylphosphinate formed by the alkylation reaction of at least one of these nitrogen atoms with the alkyl dihydrocarbylphosphinate also falls within the scope of the present invention. In the present invention, typical organic nitrogen-containing compounds include one or a combination of at least two selected from the group consisting of methylamine, ethylamine, propylamine, butylamine, pentylamine, hexylamine, heptylamine, octylamine, nonylamine, decylamine, dodecylamine, octadecylamine, cyclohexylamine, diethylamine, methylpropylamine, ethylenediamine, propylenediamine, butylenediamine, pentylenediamine, hexamethylenediamine, heptylenediamine, octylenediamine, 1,4-cyclohexanediamine, diethanolamine, m-phenylenediamine, p-phenylenediamine, piperazine, 2-methylpiperazine, 2-methylimidazole, tetraethylenepentamine, triethylenetetramine, aniline, N-methylaniline, 4-methylaniline, diaminodiphenylmethane, 2,2-bis(4-aminophenyl)propane, diaminodiphenyl sulfone, diaminobenzophenone, cyanamide, melamine and dicyandiamide.

In an embodiment, the organic nitrogen-containing compound is a primary amine compound.

In the ammonium dihydrocarbylphosphinate, at least one nitrogen atom among the nitrogen atoms that have undergone the alkylation reaction has an H atom. After the reaction between the alkyl dihydrocarbylphosphinate and the organic nitrogen-containing compound is completed, at least one H atom remains on at least one nitrogen atom participated in the alkylation reaction in the organic nitrogen moiety of the resulting ammonium dihydrocarbylphosphinate.

In an embodiment, the ammonium dihydrocarbylphosphinate has any one of the following structures: 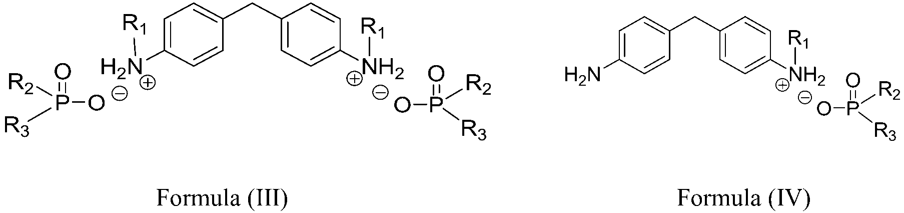 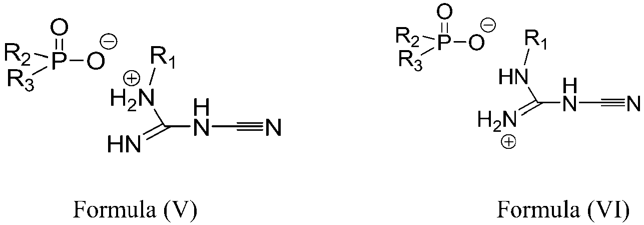 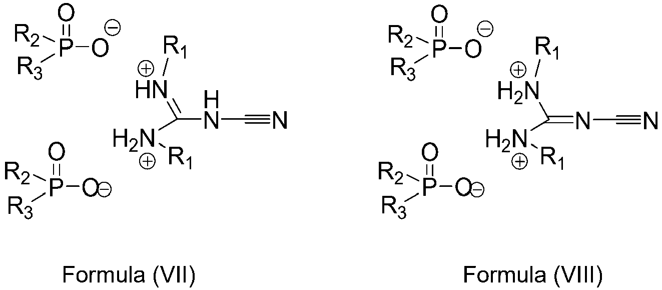 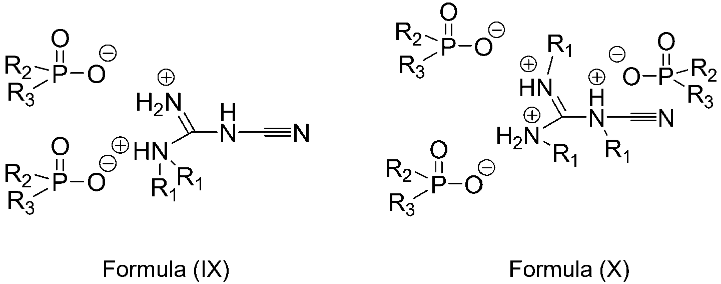 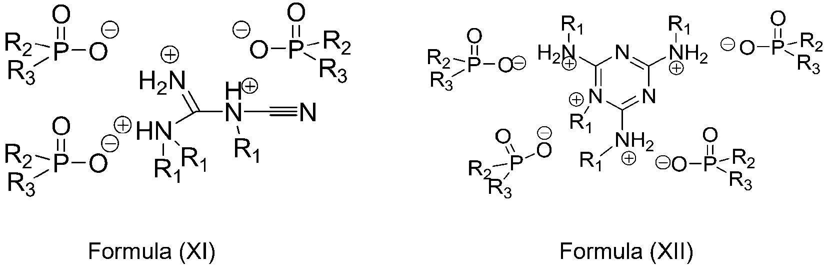 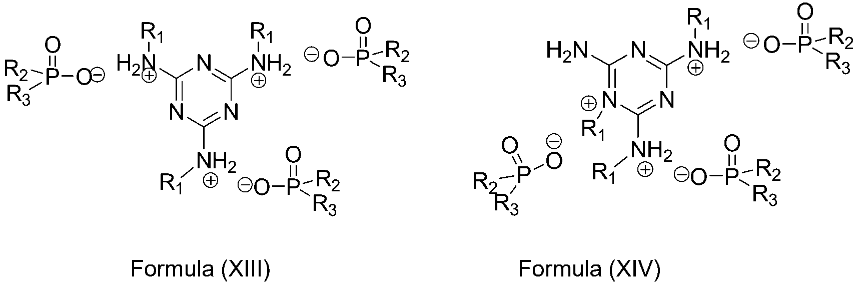 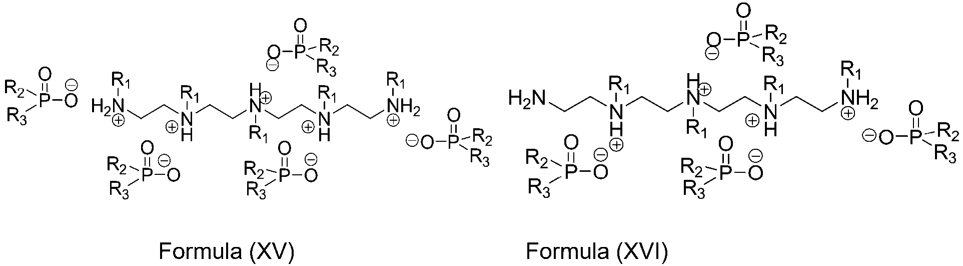 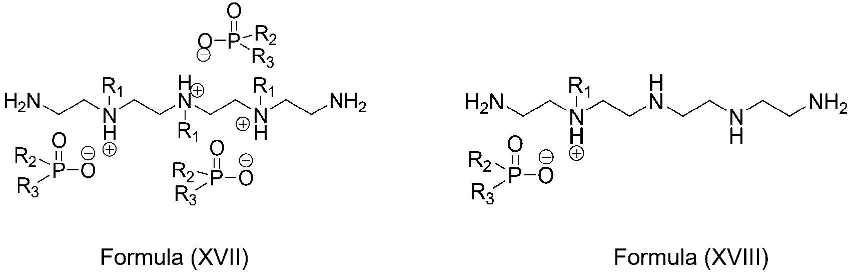 where in formulas (III) to (XVIII) above, R₁, R₂ and R₃ have the same definitions as R₁, R₂ and R₃ in formula (I).

In an embodiment, in formulas (III) to (XVIII) above, R₁ is selected from C₁~C₆ alkyl groups; R₂ and R₃ are independently selected from C₁~C₁₈ straight-chain or branched alkyl groups or C₆∼C₂₄ aryl groups.

In an embodiment, R₁ is independently selected from the group consisting of methyl, ethyl, propyl, isopropyl, and butyl; R₂ and R₃ are independently selected from the group consisting of methyl, ethyl, propyl, isopropyl, butyl, and phenyl.

In an embodiment, R₁ is selected from the group consisting of methyl, ethyl, and propyl; R₂ and R₃ are independently selected from the group consisting of ethyl, propyl, isopropyl, and butyl.

In an embodiment, R₁ is selected from the group consisting of methyl, ethyl, and propyl; R₂ and R₃ are ethyl.

In another aspect, the present invention provides a method for preparing the ammonium dihydrocarbylphosphinate as described above, including the following step:
subjecting an alkyl dihydrocarbylphosphinate and nitrogen on at least one non-tertiary amine structure in NH₃ or an organic nitrogen-containing compound to an alkylation reaction to obtain the ammonium dihydrocarbylphosphinate.

When the organic nitrogen-containing compound contains multiple nitrogen atoms, one or more of these nitrogen atoms can participate in the alkylation reaction, but at least one of the nitrogen atoms will retain at least one hydrogen atom after participating in the alkylation reaction. For example, the organic nitrogen-containing compounds used in the present invention can include primary amines, secondary amines, and imines that contain tertiary amine structures. In this case, the nitrogen in the tertiary amine structure can also be alkylated by the alkyl dihydrocarbylphosphinate, but at least one of all the nitrogen atoms in the primary amine, secondary amine, and imine that participate in the alkylation reaction retains at least one hydrogen atom.

Typical alkyl dihydrocarbylphosphinates include one or a combination of at least two selected from the group consisting of methyl dimethylphosphinate, methyl diethylphosphinate, methyl dipropylphosphinate, methyl ethylpropylphosphinate, methyl diphenylphosphinate, ethyl dimethylphosphinate, ethyl diethylphosphinate, ethyl dipropylphosphinate, ethyl ethylpropylphosphinate, propyl dimethylphosphinate, propyl diethylphosphinate, propyl dipropylphosphinate, propyl ethylpropylphosphinate, methyl methylphenylphosphinate, and methyl diphenylphosphinate.

Optionally, the organic nitrogen-containing compound includes one or a combination of at least two selected from the group consisting of methylamine, ethylamine, propylamine, butylamine, pentylamine, hexylamine, heptylamine, octylamine, nonylamine, decylamine, dodecylamine, octadecylamine, cyclohexylamine, diethylamine, methylpropylamine, ethylenediamine, propylenediamine, butylenediamine, pentylenediamine, hexamethylenediamine, heptylenediamine, octylenediamine, 1,4-cyclohexanediamine, diethanolamine, m-phenylenediamine, p-phenylenediamine, piperazine, 2-methylpiperazine, 2-methylimidazole, tetraethylenepentamine, triethylenetetramine, aniline, N-methylaniline, 4-methylaniline, diaminodiphenylmethane, 2,2-bis(4-aminophenyl)propane, diaminodiphenyl sulfone, diaminobenzophenone, cyanamide, melamine, and dicyandiamide.

In the present invention, the molar ratio of the organic nitrogen-containing compound to the alkyl dihydrocarbylphosphinate in the reaction is determined by the total number of nitrogen atoms in the organic nitrogen-containing compound.

In an embodiment, the molar ratio of the total moles of nitrogen in the organic nitrogen-containing compound to the alkyl dihydrocarbylphosphinate is 10/1 - 1/10, for example, 10/1, 9/1, 8/1, 7/1, 6/1, 5/1, 4/1, 3/1, 2/1, 1/1, 1/2, 1/3, 1/4, 1/5, 1/6, 1/7, 1/8, 1/9, or 1/10. If the ratio is too small, the flame retardancy of the resulting product will be poor; if the ratio is too high, the excess alkyl dihydrocarbylphosphinate needs to be recovered, leading to reduced economic efficiency.

In an embodiment, the molar ratio of the total moles of nitrogen in the organic nitrogen-containing compound to the alkyl dihydrocarbylphosphinate is 6/1 - 1/6; for example, it may be 5/1 - 1/6, 4/1 - 1/6, 3/1 - 1/6, 2/1 - 1/6, 1/1 - 1/6, 1/1 - 1/5, 1/1 - 1/4, 1/1 - 1/3, 1/1 - 1/2.5, or 1/1 - 1/2.

In an embodiment, the temperature of the alkylation reaction is 0 °C-250 °C, for example, 0 °C, 10 °C, 50 °C, 80 °C, 100 °C, 150 °C, 180 °C, 200 °C, 220 °C, or 250 °C.

In an embodiment, the duration of the alkylation reaction is 0.1 hours-30 hours, for example, 0.1 hours, 3 hours, 5 hours, 8 hours, 10 hours, 13 hours, 15 hours, 18 hours, 20 hours, 23 hours, 25 hours, 28 hours, or 30 hours.

In the present invention, the alkylation reaction can be carried out under normal pressure, negative pressure, or positive pressure.

When the organic nitrogen-containing compound contains 2 or more reactive nitrogen atoms, the product can be products where all or part of the nitrogen atoms are alkylated, or a mixture of these products. For example, after the reaction between diaminodiphenylmethane and methyl diethylphosphinate, the obtained products can be the compounds represented by structural formulas (III) and (IV) respectively, or a mixture of the two.

In another aspect, the present invention provides a flame-retardant polymer material, which includes a flame retardant and a polymer material, wherein the flame retardant includes the ammonium dihydrocarbylphosphinate as described above.

In an embodiment, the flame-retardant polymer material includes 0.1 wt%-50 wt% of the flame retardant and 50 wt%-99.9 wt% of the polymer material.

In the present invention, the mass content of the flame retardant in the flame-retardant polymer material is independently selected from any value or any range between any two values of 0.1%, 0.2%, 0.5%, 0.8%, 1%, 1.2%, 1.5%, 2%, 2.2%, 2.5%, 3%, 3.5%, 4%, 5%, 6%, 8%, 10%, 15%, 20%, 30%, 40%, or 50%.

In an embodiment, the flame-retardant polymer material further includes functional additives.

In an embodiment, the functional additive is at least one selected from the group consisting of reinforcing agents, anti-dripping agents, stabilizers, pigments, dyes, char-forming catalysts, dispersants, nucleating agents, inorganic fillers, organic fillers, and antioxidants.

In an embodiment, the reinforcing agent is selected from glass fibers.

In an embodiment, the anti-dripping agent is selected from Teflon.

In an embodiment, the inorganic filler is at least one selected from the group consisting of silicon dioxide, mica, calcium carbonate, aluminum oxide, aluminum nitride, talc, calcium oxide, calcium silicate, glass fiber powder, and silica.

In an embodiment, the organic filler is at least one selected from the group consisting of polytetrafluoroethylene powder, polyphenylene sulfide powder, and polyethersulfone powder.

In an embodiment, the mass content of the functional additives in the flame-retardant polymer material is 5%-40%; for example, 5%, 8%, 10%, 15%, 18%, 20%, 25%, 28%, 30%, 33%, 35%, 38%, or 40%.

In an embodiment, the flame-retardant polymer material further includes a flame retardant Q.

In an embodiment, the flame retardant Q is at least one selected from the group consisting of phosphorus-based flame retardants, nitrogen-based flame retardants, and boron-based flame retardants.

In an embodiment, the phosphorus-based flame retardant is at least one selected from the group consisting of bisphosphates, phosphazenes, dihydrocarbylphosphinates, 9,10-dihydro-9-oxa-10-phosphaphenanthrene-10-oxide derivatives (e.g., 6H-dibenzo[c,e][1,2]oxophosphorylglyceride, 6,6'-(1,2-ethanediyl)bis-6,6'-dioxide, and HTP-6123 from Guizhou Yuanyi Group), and phosphine oxide compounds.

In an embodiment, the diphosphate is at least one selected from the group consisting of bisphenol A bis(diphenyl phosphate), resorcinol bis(diphenyl phosphate) and 1,3-phenylene tetrakis(2,6-dimethylphenyl) phosphate.

In an embodiment, the phosphazene-based flame retardant is at least one selected from the group consisting of hexaphenoxycyclotriphosphazene, cyanophenoxyphosphazene (e.g., FP-300 from Fushimi Pharmaceutical Co., Ltd., Japan), allylcyclophosphazene (e.g., SPV-100 from Otsuka Chemical Co., Ltd., Japan), and cyclic biphenoxyphosphazene (e.g., BP-PZ from Otsuka Chemical Co., Ltd., Japan).

In an embodiment, the dihydrocarbylphosphinate is at least one selected from the group consisting of aluminum diethylphosphinate, aluminum diisobutylphosphinate, aluminum dipropylphosphinate, and zinc diethylphosphinate.

In an embodiment, the 9,10-dihydro-9-oxa-10-phosphaphenanthrene-10-oxide derivative is at least one selected from the group consisting of 6H-dibenzo[c,e][1,2]oxaphosphinin-6,6'-(1,4-ethanediyl)bis-6,6'-dioxide, and 6H-dibenzo[c,e][1,2]oxaphosphinin-6,6'-(1,4-phenylethanediyl)bis-6,6'-dioxide.

In an embodiment, the phosphine oxide compound is p-xylylene bis(diphenylphosphine oxide) (e.g., PQ-60 from Jinyi Chemical Industry Co., Ltd.).

In an embodiment, the nitrogen-based flame retardant is at least one selected from the group consisting of melamine cyanurate, melamine polyphosphate, and ammonium polyphosphate.

In an embodiment, the boron-based flame retardant is selected from zinc borate.

In an embodiment, the mass content of the flame retardant Q in the flame-retardant polymer material is 0.5%-20%, for example, 0.5%, 0.8%, 1%, 3%, 5%, 8%, 10%, 13%, 15%, 18%, or 20%.

In an embodiment, the polymer material is selected from the group consisting of thermoplastic polymer resins and thermosetting resins.

In an embodiment, the thermoplastic polymer resin is at least one selected from the group consisting of polybutadiene, polyamide, polyester, polyphenylene oxide, acrylonitrile-butadiene-styrene copolymer, polyethylene, polypropylene, polyvinyl chloride, polystyrene, polyimide, polyphenylene sulfide, and polyether ether ketone.

In an embodiment, the thermosetting resin is at least one selected from the group consisting of vinyl polyphenylene oxide resin, epoxy resin, cyanate ester resin, BT resin, bismaleimide resin, phenolic resin, polyurethane resin, thermosetting polyimide, unsaturated polyester resin, vinyl resin, benzoxazine resin, arylacetylene resin, and furan resin.

In an embodiment, the thermosetting resin is at least one selected from the group consisting of vinyl polyphenylene oxide resin, epoxy resin, cyanate ester resin, BT resin, bismaleimide resin, and thermosetting polyimide.

In an embodiment, the thermosetting resin is an epoxy resin.

In an embodiment, the epoxy resin is at least one selected from the group consisting of bisphenol A epoxy resin, bisphenol F epoxy resin, dicyclopentadiene epoxy resin, biphenyl epoxy resin, or naphthol epoxy resin.

The epoxy resins described in the present invention include, but are not limited to, existing epoxy resins available on the market.

In an embodiment, the flame-retardant polymer material described in the present invention is a flame-retardant epoxy resin material, which includes a flame retardant and an epoxy resin, wherein the flame retardant includes the ammonium dihydrocarbylphosphinate as described above.

In an embodiment, the flame-retardant epoxy resin material includes 0.1 wt%-18 wt% of the ammonium dihydrocarbylphosphinate and 82 wt%-99.9 wt% of the epoxy resin.

In an embodiment, the content of the flame retardant with the structure of formula (I) in the flame-retardant material is any value or any range between any two values of 0.1%, 0.2%, 0.5%, 0.8%, 1%, 1.2%, 1.5%, 2%, 2.2%, 2.5%, 3%, 3.5%, 4%, 5%, 6%, 8%, 10%, 15%, or 18% by weight.

In an embodiment, the flame-retardant epoxy resin material further includes functional additives.

In an embodiment, the functional additive is at least one selected from the group consisting of reinforcing agents, anti-dripping agents, stabilizers, pigments, dyes, char-forming catalysts, dispersants, nucleating agents, inorganic fillers, organic fillers, or antioxidants.

In an embodiment, the reinforcing agent is selected from glass fibers.

In an embodiment, the anti-dripping agent is selected from Teflon.

In an embodiment, the inorganic filler is at least one selected from the group consisting of silicon dioxide, mica, calcium carbonate, aluminum oxide, aluminum nitride, talc, calcium oxide, calcium silicate, glass fiber powder, and silica.

In an embodiment, the organic filler is at least one selected from the group consisting of polytetrafluoroethylene powder, polyphenylene sulfide powder, or polyethersulfone powder.

In an embodiment, the mass content of the functional additives in the flame-retardant epoxy resin material is 5%-40%, for example, 5%, 8%, 10%, 15%, 18%, 20%, 25%, 28%, 30%, 33%, 35%, 38%, or 40%.

The ammonium dihydrocarbylphosphinate prepared in the present invention is prepared through a simple process and is suitable for large-scale industrial production. When applied to epoxy resins, it exhibits high flame retardant efficiency and can effectively maintain the physical properties of the epoxy resins.

Compared with the prior art, the present invention has the following advantages.

The ammonium dihydrocarbylphosphinate described in the present invention has a lower melting point than the solid amine used in its preparation, is easy to liquefy, and exhibits good compatibility and fast compatibility speed with resins, enabling convenient operation. It not only overcomes the problems of solid amines such as high melting points, difficulty in liquefaction, and poor compatibility with resin systems, but also solves the corrosion problem caused by dialkylphosphinic acid or its residues.

When used as a flame retardant, the ammonium dihydrocarbylphosphinate of the present invention not only has high flame retardant efficiency but also can effectively maintain the physical properties of polymer materials, especially the glass transition temperature.

The ammonium dihydrocarbylphosphinate of the present invention is prepared through a simple process: it requires no solvent or catalyst, follows a "1+1=2" reaction pattern, has high atom economy, and is suitable for large-scale production.

Other aspects will become apparent upon reading and understanding the drawings and detailed description.

### Brief Description of the Drawings

The drawings are provided to further understand the technical solutions of this document, and constitute a part of the specification. Together with the embodiments of the present invention, they are used to explain the technical solutions of this document, and do not limit the technical solutions of this document.
Figure 1 is the ¹H NMR spectrum of DDM-MDP-1-2.
Figure 2 is the ¹H NMR spectrum of melamine-MDP-1-3.
Figure 3 is the ¹H NMR spectrum of TEPA-MDP-1-5.

### Detailed Description of Embodiments

The technical solutions of the present invention will be further illustrated below through specific examples. It should be clear to those skilled in the art that the following examples are only for facilitating the understanding of the present invention and shall not be deemed as specific limitations on the present invention.

The raw materials used in the examples are as follows.

Melamine: Sinopharm Chemical Reagent Co., Ltd.

Dicyandiamide (DICY): Shanghai Aladdin Biochemical Technology Co., Ltd.

Diaminodiphenylmethane (DDM): Shanghai Aladdin Biochemical Technology Co., Ltd.

Tetraethylenepentamine (TEPA): Shanghai Macklin Biochemical Technology Co., Ltd.

Methyl diethylphosphinate (MDP): Jiangsu Liside New Materials Co., Ltd.

Epoxy resin Type E44 (DGEBA): Baling Petrochemical Co., Ltd.

Phenolic epoxy resin SQPN-O48 (F48): Shandong Shengquan Chemical Industry Co., Ltd.

2-Methylimidazole: Saan Chemical Technology (Shanghai) Co., Ltd.

N,N-Dimethylformamide (DMF): Shanghai Aladdin Biochemical Technology Co., Ltd.

### Combustion test standard: GB/T 2408-2008

### Example 1 Preparation of the Product from Melamine and Methyl Diethylphosphinate at a Molar Ratio of 1:4

Melamine and methyl diethylphosphinate reacted at a molar ratio of 1:4, with the specific reaction procedure as follows:
2 g (0.016 mol) of melamine (melting point: 354 °C) and 13.9 g (0.102 mol) of methyl diethylphosphinate were mixed, and reacted at 200 °C for 6 h. Excess methyl diethylphosphinate was removed by vacuum distillation, and the residue was vacuum-dried at 160 °C to obtain 10.5 g (98.9%) of a yellow viscous liquid. This product is designated as melamine-MDP-1-4. ¹H NMR (solvent: DMSO-*d*₆): 2.6-3.6 ppm (N-CH₃, 12H), 1.3-2.0 ppm (-CH₂CH₃, 16H), 0.8-1.2 ppm (-CH₂CH₃, 24H). It has the following structure:

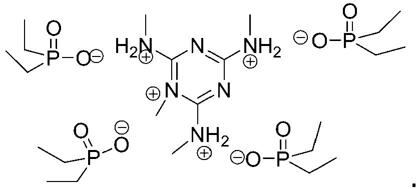

### Example 2 Preparation of the Product from Melamine and Methyl Diethylphosphinate at a Molar Ratio of 1:3

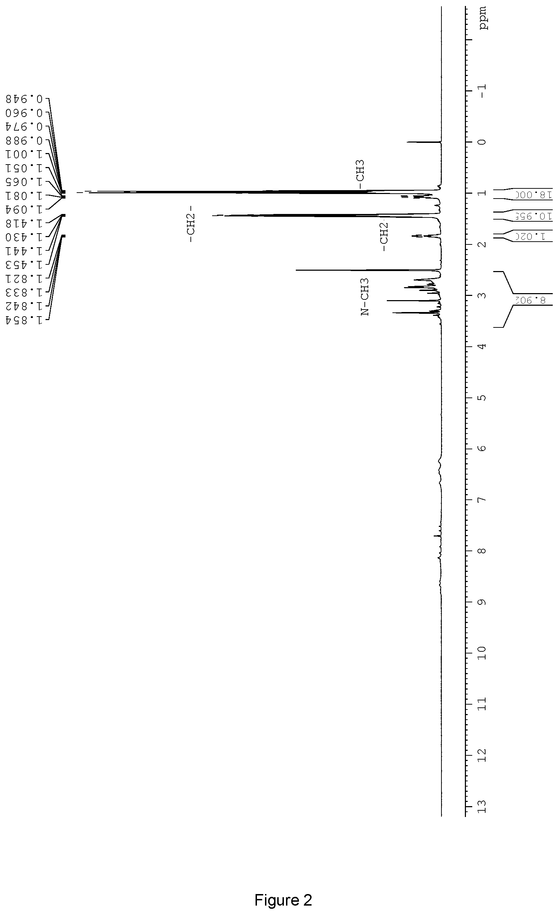

Melamine and methyl diethylphosphinate reacted at a molar ratio of 1:3, with the specific reaction procedure as follows:
10 g (0.08 mol) of melamine (melting point: 354 °C) and 32.35 g (0.24 mol) of methyl diethylphosphinate were mixed, and reacted at 200 °C for 6 h to obtain 36 g of a light yellow liquid (85.0%, attributed to transfer loss). This product is designated as melamine-MDP-1-3. The ¹H NMR spectrum of the product is shown in Figure 2. ¹H NMR (solvent: DMSO-*d*₆): 2.6-3.6 ppm (N-CH₃, 9H), 1.3-2.0 ppm (-CH₂CH₃, 12H), 0.8-1.2 ppm (-CH₂CH₃, 18H). It has the following structure:

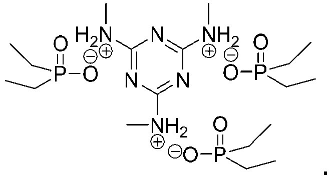

### Example 3 Preparation of the Product from Dicyandiamide and Methyl Diethylphosphinate at a Molar Ratio of 1:2

Dicyandiamide and methyl diethylphosphinate reacted at a molar ratio of 1:2, with the specific reaction procedure as follows:
5 g (0.06 mol) of dicyandiamide (209.5 °C) and 17.8 g (0.13 mol) of methyl diethylphosphinate were mixed, and reacted at 180-190 °C for 4 h. After vacuum drying at 160 °C, 18.3 g of a yellow transparent liquid was obtained (86.4%, attributed to transfer loss). This product is designated as DICY-MDP-1-2. ¹H NMR (solvent: DMSO-*d*₆): 2.6-3.7 ppm (N-CH₃, 6H), 1.3-1.5 ppm (-CH₂CH₃, 8H), 0.8-1.1 ppm (-CH₂CH₃, 12H). It has the following structure: a mixture of

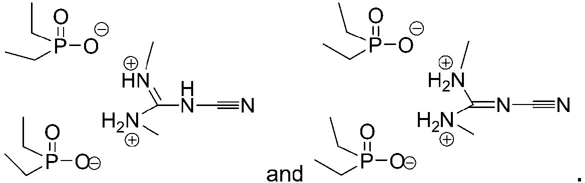

### Example 4 Preparation of the Product from Dicyandiamide and Methyl Diethylphosphinate at a Molar Ratio of 1:3

Dicyandiamide and methyl diethylphosphinate reacted at a molar ratio of 1:3, with the specific reaction procedure as follows:
5 g (0.06 mol) of dicyandiamide (209.5 °C) and 26.7 g (0.2 mol) of methyl diethylphosphinate were mixed, and reacted at 190 °C for 6 h. After vacuum drying at 160 °C, 27.1 g of a yellow transparent liquid was obtained (92.6%, attributed to transfer loss). This product is designated as DICY-MDP-1-3. ¹H NMR (solvent: DMSO-*d*₆): 2.6-3.7 ppm (N-CH₃, 9H), 1.3-1.5 ppm (-CH₂CH₃, 12H), 0.8-1.1 ppm (-CH₂CH₃, 18H). It has the following structure:

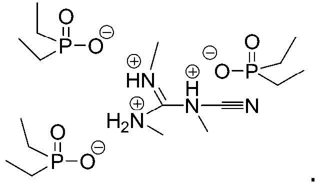

### Example 5 Preparation of the Product from Diaminodiphenylmethane and Methyl Diethylphosphinate at a Molar Ratio of 1:2

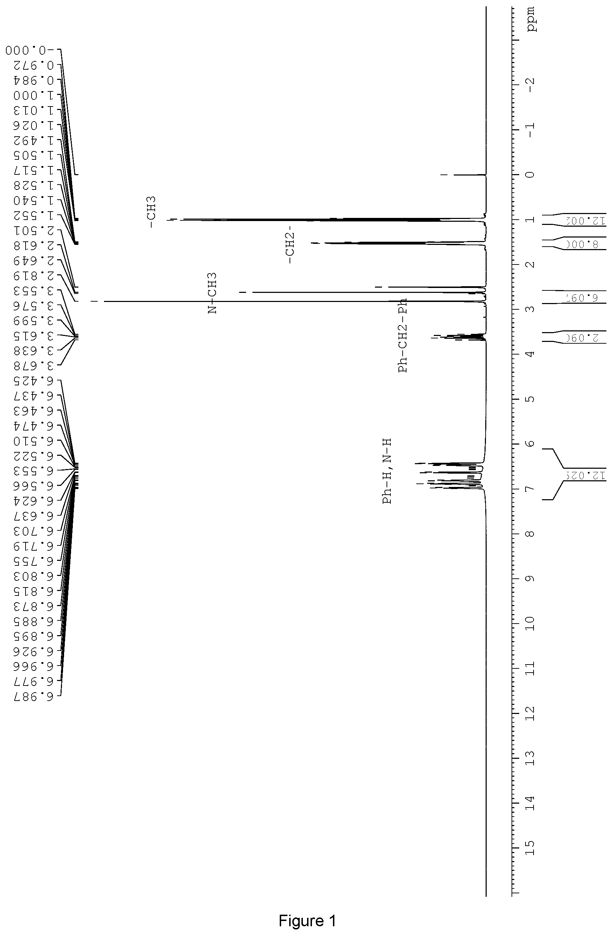

4,4'-Diaminodiphenylmethane and methyl diethylphosphinate reacted at a molar ratio of 1:2, with the specific reaction procedure as follows:
10 g (0.05 mol) of 4,4'-diaminodiphenylmethane (melting point: 89-91 °C) and 13.31 g (0.10 mol) of methyl diethylphosphinate were mixed, heated at 160 °C, and reacted for approximately 1.5 h to obtain 22.84 g (98.0%) of a wine-red liquid. This product is designated as DDM-MDP-1-2. The ¹H NMR spectrum of the product is shown in Figure 1. ¹H NMR (solvent: DMSO-*d*₆): 6.3-7.2 ppm (Ph-H, N-H, 12H), 3.6 ppm (Ph-CH₂-Ph, 2H), 2.8-3.0 ppm (N-CH₃, 6H), 1.7 ppm (-CH₂CH₃, 8H), 1.2 ppm (-CH₂CH₃, 12H). It has the following structure:

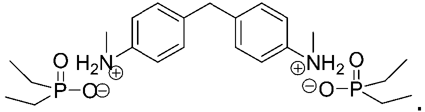

### Example 6 Preparation of the Product from Diaminodiphenylmethane and Ethyl Diethylphosphinate at a Molar Ratio of 1:2

Preparation of ethyl diethylphosphinate: 100 g (0.6 mol) of triethyl orthoacetate and 75.2 g (0.6 mol) of diethylphosphinic acid were mixed, reacted at 100-110 °C for 20 h, and then distilled at 100 °C to remove by-products, yielding 88 g (97.8%) of ethyl diethylphosphinate (EDP). ¹H NMR (solvent: CDCl₃): 3.9 ppm (P-O-CH₂CH₃, 2H), 1.6 ppm (-CH₂CH₃, 4H), 1.2 ppm (P-O-CH₂CH₃, 3H), 1.1 ppm (-CH₂CH₃, 6H). It has the following structure:

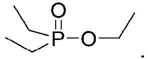

4,4'-Diaminodiphenylmethane and ethyl diethylphosphinate reacted at a molar ratio of 1:2, with the specific reaction procedure as follows: 10 g (0.05 mol) of 4,4'-diaminodiphenylmethane (melting point: 89-91 °C) and 14.82 g (0.10 mol) of ethyl diethylphosphinate were mixed, heated at 200 °C, and reacted for approximately 3 h to obtain 23.98 g (97.0%) of a wine-red liquid. This product is designated as DDM-EDP-1-2. ¹H NMR (solvent: DMSO-*d*₆): 6.3-7.2 ppm (Ph-H, 8H), 3.6 ppm (Ph-CH₂-Ph, 2H), 2.8-3.4 ppm (N-CH₂CH₃, 4H), 1.7 ppm (-CH₂CH₃, 8H), 1.2 ppm (-CH₂CH₃, N-CH₂CH₃, 18H). It has the following structure:

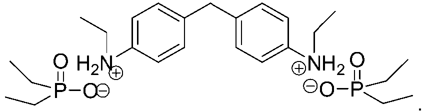

### Example 7 Preparation of the Product from Tetraethylenepentamine and Methyl Diethylphosphinate at a Molar Ratio of 1:5

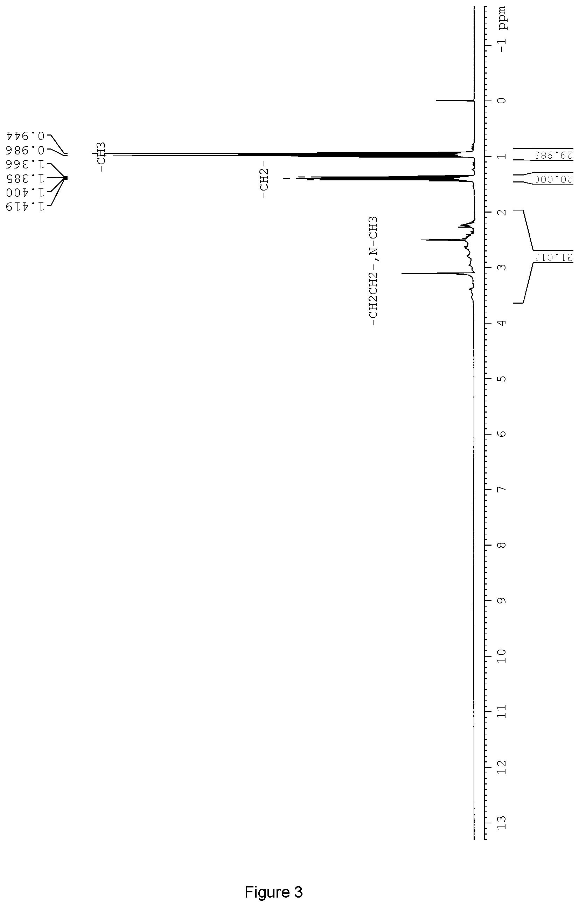

Tetraethylenepentamine and methyl diethylphosphinate reacted at a molar ratio of 1:5, with the specific reaction procedure as follows:
5 g (0.026 mol) of tetraethylenepentamine (melting point: -40 °C) and 17.4 g (0.128 mol) of methyl diethylphosphinate were mixed, reacted at 150 °C for 1 h, and then vacuum-dried at 160 °C to obtain 20.28 g of a yellow transparent liquid (91.9%, attributed to transfer loss). This product is designated as TEPA-MDP-1-5. The ¹H NMR spectrum of the product is shown in Figure 3. ¹H NMR (solvent: DMSO-*d*₆): 2.0-4.0 ppm (N-CH₃, -CH₂CH₂-, 31H), 1.3-1.5 ppm (-CH₂CH₃, 20H), 0.8-1.1 ppm (-CH₂CH₃, 30H). It has the following structure:

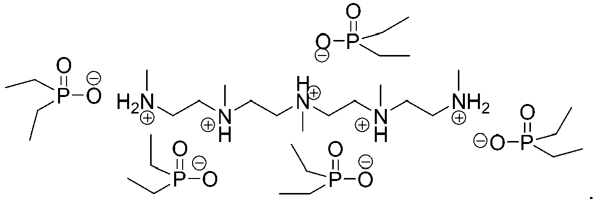

### Example 8

79.6 parts of epoxy resin DGEBA were heated at 80 °C. After the resin was fully softened, 17.4 parts of 4,4'-diaminodiphenylmethane (DDM) were added, and rapidly stirred to promote the dissolution of DDM. After most of the DDM was dissolved, 3 parts of melamine-MDP-1-4 (prepared in Example 1) were added, and stirred continuously for 1 min to form a uniform mixed solution. The mixed solution was poured into a mold preheated to 80 °C while it was still hot. After standing for 10 min, the temperature was raised to 120 °C for pre-curing for 2 h, and then further raised to 170 °C for curing for 4 h. After natural cooling to room temperature, an EP/melamine-MDP-1-4 composite material with an addition amount of 3 wt% was obtained. The thickness of the sample was 3.2 mm. It reached the UL94 V0 rating, and its Tg was 151 °C. The results are shown in Table 1.

### Example 9

80.4 parts of epoxy resin DGEBA were heated at 80 °C. After the resin was fully softened, 17.6 parts of 4,4'-diaminodiphenylmethane (DDM) were added, and rapidly stirred to promote the dissolution of DDM. After most of the DDM was dissolved, 2 parts of melamine-MDP-1-4 (prepared in Example 1) were added, and stirred continuously for 1 min to form a uniform mixed solution. The mixed solution was poured into a mold preheated to 80 °C while it was still hot. After standing for 10 min, the temperature was raised to 120 °C for pre-curing for 2 h, and then further raised to 170 °C for curing for 4 h. After natural cooling to room temperature, an EP/melamine-MDP-1-4 composite material with an addition amount of 2 wt% was obtained. The thickness of the sample was 3.2 mm. It reached the UL94 V1 rating. The results are shown in Table 1.

### Example 10

79.6 parts of epoxy resin DGEBA were heated at 80 °C. After the resin was fully softened, 17.4 parts of 4,4'-diaminodiphenylmethane (DDM) were added, and rapidly stirred to promote the dissolution of DDM. After most of the DDM was dissolved, 3 parts of melamine-MDP-1-3 (prepared in Example 2) were added, and stirred continuously for 1 min to form a uniform mixed solution. The mixed solution was poured into a mold preheated to 80 °C while it was still hot. After standing for 10 min, the temperature was raised to 120 °C for pre-curing for 2 h, and then further raised to 170 °C for curing for 4 h. After natural cooling to room temperature, an EP/melamine-MDP-1-3 composite material with an addition amount of 3 wt% was obtained. The thickness of the sample was 3.2 mm. It reached the UL94 V0 rating, and its Tg was 150 °C. The results are shown in Table 1.

### Example 11

80.4 parts of epoxy resin DGEBA were heated at 80 °C. After the resin was fully softened, 17.6 parts of 4,4'-diaminodiphenylmethane (DDM) were added, and rapidly stirred to promote the dissolution of DDM. After most of the DDM was dissolved, 2 parts of melamine-MDP-1-3 (prepared in Example 2) were added, and stirred continuously for 1 min to form a uniform mixed solution. The mixed solution was poured into a mold preheated to 80 °C while it was still hot. After standing for 10 min, the temperature was raised to 120 °C for pre-curing for 2 h, and then further raised to 170 °C for curing for 4 h. After natural cooling to room temperature, an EP/melamine-MDP-1-3 composite material with an addition amount of 2 wt% was obtained. The thickness of the sample was 3.2 mm. It reached the UL94 V1 rating. The results are shown in Table 1.

### Example 12

79.6 parts of epoxy resin DGEBA were heated at 80 °C. After the resin was fully softened, 17.4 parts of 4,4'-diaminodiphenylmethane (DDM) were added, and rapidly stirred to promote the dissolution of DDM. After most of the DDM was dissolved, 3 parts of DICY-MDP-1-2 (prepared in Example 3) were added, and stirred continuously for 1 min to form a uniform mixed solution. The mixed solution was poured into a mold preheated to 80 °C while it was still hot. After standing for 10 min, the temperature was raised to 120 °C for pre-curing for 2 h, and then further raised to 170 °C for curing for 4 h. After natural cooling to room temperature, an EP/DICY-MDP-1-2 composite material with an addition amount of 3 wt% was obtained. The thickness of the sample was 3.2 mm. It reached the UL94 V0 rating, and its Tg was 147 °C. The results are shown in Table 1.

### Example 13

80.4 parts of epoxy resin DGEBA were heated at 80 °C. After the resin was fully softened, 17.6 parts of 4,4'-diaminodiphenylmethane (DDM) were added, and rapidly stirred to promote the dissolution of DDM. After most of the DDM was dissolved, 2 parts of DICY-MDP-1-2 (prepared in Example 3) were added, and stirred continuously for 1 min to form a uniform mixed solution. The mixed solution was poured into a mold preheated to 80 °C while it was still hot. After standing for 10 min, the temperature was raised to 120 °C for pre-curing for 2 h, and then further raised to 170 °C for curing for 4 h. After natural cooling to room temperature, an EP/DICY-MDP-1-2 composite material with an addition amount of 2 wt% was obtained. The thickness of the sample was 3.2 mm. It reached the UL94 V1 rating. The results are shown in Table 1.

### Example 14

80.4 parts of epoxy resin DGEBA were heated at 80 °C. After the resin was fully softened, 17.6 parts of 4,4'-diaminodiphenylmethane (DDM) were added, and rapidly stirred to promote the dissolution of DDM. After most of the DDM was dissolved, 2 parts of DICY-MDP-1-3 (prepared in Example 4) were added, and stirred continuously for 1 min to form a uniform mixed solution. The mixed solution was poured into a mold preheated to 80 °C while it was still hot. After standing for 10 min, the temperature was raised to 120 °C for pre-curing for 2 h, and then further raised to 170 °C for curing for 4 h. After natural cooling to room temperature, an EP/DICY-MDP-1-3 composite material with an addition amount of 2 wt% was obtained. The thickness of the sample was 3.2 mm. It reached the UL94 V0 rating, and its Tg was 153 °C. The results are shown in Table 1.

### Example 15

81.3 parts of epoxy resin DGEBA were heated at 80 °C. After the resin was fully softened, 17.7 parts of 4,4'-diaminodiphenylmethane (DDM) were added, and rapidly stirred to promote the dissolution of DDM. After most of the DDM was dissolved, 1 part of DICY-MDP-1-3 (prepared in Example 4) was added, and stirred continuously for 1 min to form a uniform mixed solution. The mixed solution was poured into a mold preheated to 80 °C while it was still hot. After standing for 10 min, the temperature was raised to 120 °C for pre-curing for 2 h, and then further raised to 170 °C for curing for 4 h. After natural cooling to room temperature, an EP/DICY-MDP-1-3 composite material with an addition amount of 1 wt% was obtained. The thickness of the sample was 3.2 mm. It reached the UL94 V1 rating. The results are shown in Table 1.

### Example 16

78.8 parts of epoxy resin DGEBA were heated at 80 °C. After the resin was fully softened, 17.2 parts of 4,4'-diaminodiphenylmethane (DDM) were added, and rapidly stirred to promote the dissolution of DDM. After most of the DDM was dissolved, 4 parts of DDM-MDP-1-2 (prepared in Example 5) were added, and stirred continuously for 1 min to form a uniform mixed solution. The mixed solution was poured into a mold preheated to 80 °C while it was still hot. After standing for 10 min, the temperature was raised to 120 °C for pre-curing for 2 h, and then further raised to 170 °C for curing for 4 h. After natural cooling to room temperature, an EP/DDM-MDP-1-2 composite material with an addition amount of 4 wt% was obtained. The thickness of the sample was 3.2 mm. It reached the UL94 V0 rating, and its Tg was 145 °C. The results are shown in Table 1.

### Example 17

79.6 parts of epoxy resin DGEBA were heated at 80 °C. After the resin was fully softened, 17.4 parts of 4,4'-diaminodiphenylmethane (DDM) were added, and rapidly stirred to promote the dissolution of DDM. After most of the DDM was dissolved, 3 parts of DDM-MDP-1-2 (prepared in Example 5) were added, and stirred continuously for 1 min to form a uniform mixed solution. The mixed solution was poured into a mold preheated to 80 °C while it was still hot. After standing for 10 min, the temperature was raised to 120 °C for pre-curing for 2 h, and then further raised to 170 °C for curing for 4 h. After natural cooling to room temperature, an EP/DDM-MDP-1-2 composite material with an addition amount of 3 wt% was obtained. The thickness of the sample was 3.2 mm. It reached the UL94 V1 rating. The results are shown in Table 1.

### Example 18

81.0 parts of epoxy resin DGEBA were heated at 80 °C. After the resin was fully softened, 15.0 parts of 4,4'-diaminodiphenylmethane (DDM) were added, and rapidly stirred to promote the dissolution of DDM. After most of the DDM was dissolved, 4 parts of DDM-MDP-1-2 (prepared in Example 5, used to replace part of DDM based on its 4 degree of functionality) were added, and stirred continuously for 1 min to form a uniform mixed solution. The mixed solution was poured into a mold preheated to 80 °C while it was still hot. After standing for 10 min, the temperature was raised to 120 °C for pre-curing for 2 h, and then further raised to 170 °C for curing for 4 h. After natural cooling to room temperature, an EP/DDM-MDP-1-2-react-4 composite material with an addition amount of 4 wt% was obtained. It reached the UL94 V0 rating. Soxhlet extraction was performed with dichloromethane; after 24 h, no DDM-MDP-1-2 was detected in the extracting solution via ¹H NMR and ³¹P NMR. This indicates that DDM-MDP-1-2 participated in the curing process.

### Example 19

80.1 parts of epoxy resin DGEBA were heated at 80 °C. After the resin was fully softened, 16.9 parts of 4,4'-diaminodiphenylmethane (DDM) were added, and rapidly stirred to promote the dissolution of DDM. After most of the DDM was dissolved, 3 parts of DDM-MDP-1-2 (prepared in Example 2, used to replace part of DDM based on its 2 degree of functionality) were added, and stirred continuously for 1 min to form a uniform mixed solution. The mixed solution was poured into a mold preheated to 80 °C while it was still hot. After standing for 10 min, the temperature was raised to 120 °C for pre-curing for 2 h, and then further raised to 180 °C for curing for 4 h. After natural cooling to room temperature, an EP/DDM-MDP-1-2-react-2 composite material with an addition amount of 3 wt% was obtained. It reached the UL94 V0 rating, and its Tg was 150 °C. The results are shown in Table 1.

### Example 20

78.8 parts of epoxy resin DGEBA were heated at 80 °C. After the resin was fully softened, 17.2 parts of 4,4'-diaminodiphenylmethane (DDM) were added, and rapidly stirred to promote the dissolution of DDM. After most of the DDM was dissolved, 4 parts of DDM-EDP-1-2 (prepared in Example 6) were added, and stirred continuously for 1 min to form a uniform mixed solution. The mixed solution was poured into a mold preheated to 80 °C while it was still hot. After standing for 10 min, the temperature was raised to 120 °C for pre-curing for 2 h, and then further raised to 170 °C for curing for 4 h. After natural cooling to room temperature, an EP/DDM-EDP-1-2 composite material with an addition amount of 4 wt% was obtained. The thickness of the sample was 3.2 mm. It reached the UL94 V0 rating, and its Tg was 146 °C. The results are shown in Table 1.

### Example 21

79.6 parts of epoxy resin DGEBA were heated at 80 °C. After the resin was fully softened, 17.2 parts of 4,4'-diaminodiphenylmethane (DDM) were added, and rapidly stirred to promote the dissolution of DDM. After most of the DDM was dissolved, 3 parts of DDM-EDP-1-2 (prepared in Example 6) were added, and stirred continuously for 1 min to form a uniform mixed solution. The mixed solution was poured into a mold preheated to 80 °C while it was still hot. After standing for 10 min, the temperature was raised to 120 °C for pre-curing for 2 h, and then further raised to 170 °C for curing for 4 h. After natural cooling to room temperature, an EP/DDM-EDP-1-2 composite material with an addition amount of 3 wt% was obtained. The thickness of the sample was 3.2 mm. It reached the UL94 V1 rating. The results are shown in Table 1.

### Example 22

79.6 parts of epoxy resin DGEBA were heated at 80 °C. After the resin was fully softened, 17.4 parts of 4,4'-diaminodiphenylmethane (DDM) were added, and rapidly stirred to promote the dissolution of DDM. After most of the DDM was dissolved, 3 parts of TEPA-MDP-1-5 (prepared in Example 7) were added, and stirred continuously for 1 min to form a uniform mixed solution. The mixed solution was poured into a mold preheated to 80 °C while it was still hot. After standing for 10 min, the temperature was raised to 120 °C for pre-curing for 2 h, and then further raised to 170 °C for curing for 4 h. After natural cooling to room temperature, an EP/TEPA-MDP-1-5 composite material with an addition amount of 3 wt% was obtained. The thickness of the sample was 3.2 mm. It reached the UL94 V0 rating, and its Tg was 148 °C. The results are shown in Table 1.

### Example 23

80.4 parts of epoxy resin DGEBA were heated at 80 °C. After the resin was fully softened, 17.6 parts of 4,4'-diaminodiphenylmethane (DDM) were added, and rapidly stirred to promote the dissolution of DDM. After most of the DDM was dissolved, 2 parts of TEPA-MDP-1-5 (prepared in Example 7) were added, and stirred continuously for 1 min to form a uniform mixed solution. The mixed solution was poured into a mold preheated to 80 °C while it was still hot. After standing for 10 min, the temperature was raised to 120 °C for pre-curing for 2 h, and then further raised to 170 °C for curing for 4 h. After natural cooling to room temperature, an EP/TEPA-MDP-1-5 composite material with an addition amount of 2 wt% was obtained. The thickness of the sample was 3.2 mm. It reached the UL94 V1 rating. The results are shown in Table 1.

### Example 24

83.2 parts of F48 epoxy resin, 8.7 parts of dicyandiamide, 0.1 part of 2-methylimidazole, and 8 parts of DICY-MDP-1-3 (prepared in Example 4) were mixed. N,N-dimethylformamide (DMF) with the same mass as dicyandiamide was added to promote dissolution, and the mixture was heated and stirred at 100 °C. After forming a homogeneous solution, DMF was removed in a vacuum oven at 100 °C. Finally, the mixed solution was poured into a mold preheated to 100 °C, pre-cured at 130 °C for 1 h, and then cured at 170 °C for 4 h. An EP-F48/DICY-MDP-1-3 composite material with an addition amount of 8% was obtained. The thickness of the sample was 3.2 mm. It reached the UL94 V0 rating. The results are shown in Table 1.

### Example 25

83.2 parts of F48 epoxy resin, 8.7 parts of dicyandiamide, 0.1 part of 2-methylimidazole, and 8 parts of TEPA-MDP-1-5 (prepared in Example 7) were mixed. N,N-dimethylformamide (DMF) with the same mass as dicyandiamide was added to promote dissolution, and the mixture was heated and stirred at 100 °C. After forming a homogeneous solution, DMF was removed in a vacuum oven at 100 °C. Finally, the mixed solution was poured into a mold preheated to 100 °C, pre-cured at 130 °C for 1 h, and then cured at 170 °C for 4 h. An EP-F48/TEPA-MDP-1-5 composite material with an addition amount of 8% was obtained. The thickness of the sample was 3.2 mm. It reached the UL94 V0 rating. The results are shown in Table 1.

### Example 26

Polypropylene (PP) was dried in a blast oven at 80 °C for 4 h. Then, 80 parts of PP and 20 parts of DICY-MDP-1-3 (prepared in Example 4) were melted in an internal mixer at a rotation speed of 50 r/min, with the temperature set to 200 °C. After 6 minutes, the mixture was taken out for cooling and drying. Subsequently, the mixture was filled into a mold, preheated in a plate vulcanizer at 200 °C for 10 minutes, held at 10 MPa for 5 minutes, and then cold-pressed. After cooling, the sample was cut and tested. The thickness of the sample was 3.2 mm. It reached the UL94 V0 rating.

### Comparative Example 1

Reaction of 4,4'-diaminodiphenylmethane and diethylphosphinic acid at a molar ratio of 1:2: a total of 5 g (0.025 mol) of 4,4'-diaminodiphenylmethane was added in batches to 5.97 g (0.049 mol) of diethylphosphinic acid, and reacted at 80-100 °C. The reaction was deemed complete when 4,4'-diaminodiphenylmethane was fully dissolved. 10.4 g (94.8%, attributed to transfer loss) of a yellow liquid product DDM-DEP-1-2 was obtained. This product slowly crystallized at room temperature to form a yellow-green solid, which was highly hygroscopic in air, resulting in inconvenient use. ¹H NMR: 7.0 ppm (Ph-H, 4H), 6.8 ppm (N-H, 6H), 6.7 ppm (Ph-H, 4H), 3.8 ppm (Ph-CH₂-Ph, 2H), 1.7 ppm (-CH₂CH₃, 8H), 1.2 ppm (-CH₂CH₃, 12H). It has the following structure:

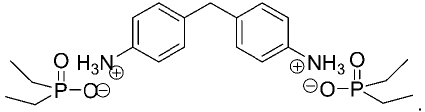

### Comparative Example 2

Reaction of melamine and diethylphosphinic acid at a molar ratio of 1:3: 5 g (0.04 mol) of melamine and 14.51 g (0.12 mol) of diethylphosphinic acid were mixed, and reacted at 190-200 °C for 2 h. 17.55 g (89.9%, attributed to transfer loss) of a light-yellow pasty solid melamine-DEP-1-3 was obtained. This solid exhibited extremely poor compatibility with DGEBA; after curing, most of the melamine-DEP-1-3 existed in the EP in a solid form. ¹H NMR: 6.0-11.0 ppm (N-H, 9H), 1.3-1.5 ppm (-CH₂CH₃, 12H), 0.8-1.1 ppm (-CH₂CH₃, 18H). It has the following structure:

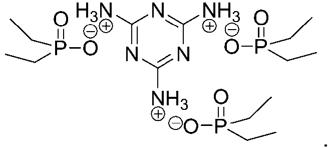

### Comparative Example 3

Reaction of dicyandiamide and diethylphosphinic acid at a molar ratio of 1:2: 5 g (0.06 mol) of dicyandiamide and 14.51 g (0.12 mol) of diethylphosphinic acid were mixed, and reacted at 100 °C. Alkaline gas was detected in the exhaust gas using pH test paper. NMR results indicated that the mixture after reaction was a mixture of dicyandiamide, diethylphosphinic acid, and the product DICY-DEP-1-2, accompanied by a large amount of by-products. Pure DICY-DEP-1-2 cannot be obtained.

### Comparative Example 4

Reaction of dicyandiamide and diethylphosphinic acid at a molar ratio of 1:2: 5 g (0.06 mol) of dicyandiamide and 14.51 g (0.12 mol) of diethylphosphinic acid were mixed and dissolved in 30 g of water. After reacting at 70 °C for 30 min, an aqueous solution of DICY-DEP-1-2 was obtained. Water was removed by rotary evaporation at 70 °C, but the product decomposed. The peak of diethylphosphinic acid reappeared in the ³¹P NMR spectrum, indicating that diethylphosphinic acid was regenerated.

### Comparative Example 5

Reaction of tetraethylenepentamine (TEPA) and diethylphosphinic acid at a molar ratio of 1:5: 5 g (0.026 mol) of tetraethylenepentamine and 15.31 g (0.12 mol) of diethylphosphinic acid were mixed and heated to 100 °C. A large amount of alkaline gas was detected in the exhaust gas using pH test paper. Pure TEPA-DEP-1-5 cannot be obtained.

### Comparative Example 6

82.1 parts of epoxy resin DGEBA were heated at 80 °C. After the resin was fully softened, 17.9 parts of 4,4'-diaminodiphenylmethane (DDM) were added, and rapidly stirred to promote the dissolution of DDM. After DDM was completely dissolved, the mixed solution was poured into a mold preheated to 80 °C while it was still hot. After standing for 10 min, the temperature was raised to 120 °C for pre-curing for 2 h, and then further raised to 170 °C for curing for 4 h. After natural cooling to room temperature, a pure EP material was obtained. The thickness of the sample was 3.2 mm. It reached the UL94 NR rating, and its Tg was 149 °C. The results are shown in Table 1.

### Comparative Example 7

79.6 parts of epoxy resin DGEBA were heated at 80 °C. After the resin was fully softened, 17.4 parts of 4,4'-diaminodiphenylmethane (DDM) were added, and rapidly stirred to promote the dissolution of DDM. After most of the DDM was dissolved, 3 parts of melamine-DEP-1-3 (prepared in Comparative Example 2) were added, and stirred continuously for 1 min to form a uniform mixed solution. The mixed solution was poured into a mold preheated to 80 °C while it was still hot. After standing for 10 min, the temperature was raised to 120 °C for pre-curing for 2 h, and then further raised to 170 °C for curing for 4 h. After natural cooling to room temperature, an EP/melamine-DEP-1-3 composite material with an addition amount of 3 wt% was obtained. The thickness of the sample was 3.2 mm. It reached the UL94 V1 rating. The results are shown in Table 1.

### Comparative Example 8

79.6 parts of epoxy resin DGEBA were heated at 80 °C. After the resin was fully softened, 17.4 parts of 4,4'-diaminodiphenylmethane (DDM) were added, and rapidly stirred to promote the dissolution of DDM. After most of the DDM was dissolved, 3 parts of DDM-DEP-1-2 (prepared in Comparative Example 1) were added, and stirred continuously for 1 min to form a uniform mixed solution. The mixed solution was poured into a mold preheated to 80 °C while it was still hot. After standing for 10 min, the temperature was raised to 120 °C for pre-curing for 2 h, and then further raised to 170 °C for curing for 4 h. After natural cooling to room temperature, an EP/DDM-DEP-1-2 composite material with an addition amount of 3 wt% was obtained. The thickness of the sample was 3.2 mm. It reached the UL94 V0 rating, and its Tg was 153 °C. The results are shown in Table 1.

### Comparative Example 9

90.4 parts of F48 epoxy resin, 9.5 parts of dicyandiamide, and 0.1 part of 2-methylimidazole were mixed. N,N-dimethylformamide (DMF) with the same mass as dicyandiamide was added to promote dissolution, and the mixture was heated and stirred at 100 °C. After forming a homogeneous solution, DMF was removed in a vacuum oven at 100 °C. Finally, the mixed solution was poured into a mold preheated to 100 °C, pre-cured at 130 °C for 1 h, and then cured at 170 °C for 4 h. A pure EP-F48 was obtained. The thickness of the sample was 3.2 mm. It reached the UL94 NR rating. The results are shown in Table 1.

### Comparative Example 10

100 parts of polypropylene (PP) were dried in a blast oven at 80 °C for 4 h. Then, the PP was melted in an internal mixer at a rotation speed of 50 r/min, with the temperature set to 200 °C. After 6 minutes, the resultant was taken out for cooling and drying. Subsequently, the PP was filled into a mold, preheated in a plate vulcanizer at 200 °C for 10 minutes, held at 10 MPa for 5 minutes, and then cold-pressed. After cooling, the sample was cut and tested. The thickness of the sample was 3.2 mm. It reached the UL94 NR rating.

In Example 18, a flame retardant prepared using DDM as the raw material was used as a reactive additive to replace part of the curing agent DDM. Soxhlet extraction was performed on the cured sample, and no flame retardant DDM-MDP-1-2 was detected in the extracting solution. This indicates that DDM-MDP-1-2 can participate in the curing of epoxy resin and can be used as a reactive flame retardant.

Both Comparative Example 7 and Examples 8-11 used flame retardants prepared with melamine as the raw material. However, the flame retardant efficiency of the product from the reaction of methyl diethylphosphinate and melamine was significantly higher than that of the neutralization product from diethylphosphinic acid and melamine (prepared in Comparative Example 2)-even though the former had a lower phosphorus content. This result is highly unexpected.

Both Comparative Example 8 and Examples 16, 17, and 19 used flame retardants prepared with DDM as the raw material. The flame retardant efficiency of the flame retardant in the present invention was comparable to that of the neutralization product from diethylphosphinic acid and DDM (prepared in Comparative Example 1). However, the latter was prone to hygroscopicity, making it inconvenient to store and use.

Examples 12-15, 22, and 23 demonstrate that the products from the reaction of diethylphosphinate esters with DICY and TEPA also exhibit extremely high flame retardant efficiency. Especially in Example 14, only 2 parts of the flame retardant in the present invention were used to achieve the V0 rating; even with 1 part (in Example 15), the epoxy resin significantly reached the V1 rating. In contrast, the corresponding neutralization products of diethylphosphinic acid cannot be obtained due to the acid hydrolysis of DICY and TEPA during the synthesis process (Comparative Examples 3, 4, and 5). This highlights the advantage of using diethylphosphinate esters instead of diethylphosphinic acid.

The comparison between Comparative Example 9 and Examples 24 and 25 shows that the flame retardant in the present invention has excellent flame retardant performance in novolac epoxy resins, and an addition amount less than 10% can meet the flame retardant requirements. Example 26 illustrates the application of the flame retardant in the present invention in polypropylene (PP): using the flame retardant prepared with DICY as the raw material, PP can pass the UL94 test with an addition amount of 20% of this flame retardant. This demonstrates the wide applicability of the flame retardant in the present invention in polymer materials such as epoxy resins and polyolefins.

**Table 1 Application of ammonium dihydrocarbylphosphinate in Epoxy Resins**

| | Epoxy Resin | Epoxy Resin Dosage /wt% | Curing Agent | Curing Agent Dosage/ wt% | Accelerator | Accelerator Dosage/ wt% | Flame Retardant | Flame Retardant Dosage/ wt% | UL94 Rating | Tg/ °C |
|---|---|---|---|---|---|---|---|---|---|---|
| Comparative Example 6 | DGEBA | 82.1 | DD M | 17.9 | | | | | NR | 149 |
| Comparative Example 7 | DGEB A | 79.6 | DDM | 17.4 | | | Product of Comparative Example 2 melamin e-DEP-1-3 | 3 | V1 | |
| Comparative Example 8 | DGEB Al | 79.6 | DD M | 17.4 | | | Product of Comparative Example 1 DDM-DEP-1-2 | 3 | vo | 153 |
| Comparative Example 9 | F48 | 90.4 | DIC Y | 9.5 | 2-Methylimidazole | 0.1 | | | NR | |
| Example 8 | DGEB A | 79.6 | DD M | 17.4 | | | Product of Example 1 melamin e-MDP-1-4 | 3 | V0 | 151 |
| Example 9 | DGEB A | 80.4 | DD M | 17.6 | | | Product of Example 1 melamin e-MDP-1-4 | 2 | V1 | |
| Example 10 | DGEBA | 79.6 | DDM | 17.4 | | | Product of Example 2 melamin e-MDP-1-3 | 3 | V0 | 150 |
| Example 11 | DGEB A | 80.4 | DD M | 17.6 | | | Product of Example 2 melamin e-MDP-1-3 | 2 | V1 | |
| Example 12 | DGEB A | 79.6 | DD M | 17.4 | | | Product of Example 3 DICY-MDP-1-2 | 3 | V0 | 147 |
| Example 13 | DGEB A | 80.4 | DD M | 17.6 | | | Product of Example 3 DICY-MDP-1-2 | 2 | V1 | |
| Example 14 | DGEB A | 80.4 | DD M | 17.6 | | | Product of Example 4 DICY-MDP-1-3 | 2 | V0 | 153 |
| Example 15 | DGEB A | 81.3 | DD M | 17.7 | | | Product of Example 4 DICY-MDP-1-3 | 1 | V1 | |
| Example 16 | DGEB A | 78.8 | DD M | 17.2 | | | Product of Example 5 DDM-MDP-1-2 | 4 | V0 | 145 |
| Example 17 | DGEB A | 79.6 | DD M | 17.4 | | | Product of Example 5 DDM-MDP-1-2 | 3 | V1 | |
| Example 19 | DGEB A | 80.1 | DD M | 16.9 | | | Product of Example 5 DDM-MDP-1-2 | 3 | V0 | 150 |
| Example 20 | DGEB A | 78.8 | DD M | 17.2 | | | Product of Example 5 DDM-EDP-1-2 | 4 | V0 | 146 |
| Example 21 | DGEB A | 79.6 | DD M | 17.4 | | | Product of Example 5 DDM-EDP-1-2 | 3 | V1 | |
| Example 22 | DGEB A | 79.6 | DD M | 17.4 | | | Product of Example 6 TEPA-MDP-1-5 | 3 | V0 | 148 |
| Example 23 | DGEB A | 80.4 | DD M | 17.6 | | | Product of Example 6 TEPA-MDP-1-5 | 2 | V1 | |
| Example 24 | F48 | 83.2 | DIC Y | 8.7 | 2-Methylimid azole | 0.1 | Product of Example 4 DICY-MDP-1-3 | 8 | V0 | |
| Example 25 | F48 | 83.2 | DIC Y | 8.7 | 2-Methylimid azole | 0.1 | Product of Example 6 TEPA-MDP-1-5 | 8 | V0 | |

The applicant states that the present invention illustrates the ammonium dihydrocarbylphosphinate of the present invention, as well as their preparation methods and applications, through the above-mentioned examples. However, the present invention is not limited to the above-mentioned examples, which means that the present invention does not necessarily rely on the above-mentioned examples for implementation. It should be clear to those skilled in the art that any improvements to the present invention, equivalent substitution of the raw materials selected in the present invention, addition of auxiliary components, selection of specific methods, etc., all fall within the protection scope and disclosure scope of the present invention.

## Claims

1. An ammonium dihydrocarbylphosphinate, **characterized in that** the ammonium dihydrocarbylphosphinate is obtained by an alkylation reaction between an alkyl dihydrocarbylphosphinate having a structural formula shown in Formula (I) and nitrogen on at least one non-tertiary amine structure in NH₃ or an organic nitrogen-containing compound, wherein an alkyl group participating in the alkylation reaction is derived from R₁ in the structural formula shown in Formula (I), the organic nitrogen-containing compound is selected from primary amines, secondary amines or imine compounds, and in the ammonium dihydrocarbylphosphinate, at least one nitrogen atom among nitrogen atoms that have undergone the alkylation reaction has both an H atom and an R₁ group obtained through the alkylation reaction: 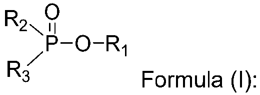
where R₁ is selected from the group consisting of C₁~C₁₈ straight-chain alkyl groups, C₁~C₁₈ branched-chain alkyl groups, and C₃~C₁₈ cycloalkyl groups; and
R₂ and R₃ are independently selected from the group consisting of C₁~C₁₈ straight-chain alkyl groups, C₁~C₁₈ branched-chain alkyl groups, C₃~C₁₈ cycloalkyl groups, and C₆∼C₂₄ aryl groups.

2. The ammonium dihydrocarbylphosphinate according to claim 1, wherein R₁ is any one selected from the group consisting of methyl, ethyl, propyl, butyl, pentyl, hexyl, heptyl, octyl, nonyl and decyl.

3. The ammonium dihydrocarbylphosphinate according to claim 1, wherein R₂ and R₃ are independently any one selected from the group consisting of methyl, ethyl, propyl, butyl, pentyl, hexyl, heptyl, octyl, nonyl, decyl and phenyl.

4. The ammonium dihydrocarbylphosphinate according to claim 1, wherein R₁ is any one selected from the group consisting of methyl, ethyl, propyl and butyl.

5. The ammonium dihydrocarbylphosphinate according to claim 1, wherein R₁ is any one selected from the group consisting of methyl and ethyl.

6. The ammonium dihydrocarbylphosphinate according to claim 1, wherein R₂ and R₃ are independently any one selected from the group consisting of ethyl, propyl and butyl.

7. The ammonium dihydrocarbylphosphinate according to claim 1, wherein R₂ and R₃ are ethyl.

8. The ammonium dihydrocarbylphosphinate according to any one of claims 1-7, wherein the alkyl dihydrocarbylphosphinate comprises one or a combination of at least two selected from the group consisting of methyl dimethylphosphinate, methyl diethylphosphinate, methyl dipropylphosphinate, methyl ethylpropylphosphinate, methyl diphenylphosphinate, ethyl dimethylphosphinate, ethyl diethylphosphinate, ethyl dipropylphosphinate, ethyl ethylpropylphosphinate, propyl dimethylphosphinate, propyl diethylphosphinate, propyl dipropylphosphinate, propyl ethylpropylphosphinate, methyl methylphenylphosphinate and methyl diphenylphosphinate;
optionally, the organic nitrogen-containing compound comprises one or a combination of at least two selected from the group consisting of methylamine, ethylamine, propylamine, butylamine, pentylamine, hexylamine, heptylamine, octylamine, nonylamine, decylamine, dodecylamine, octadecylamine, cyclohexylamine, diethylamine, methylpropylamine, ethylenediamine, propylenediamine, butylenediamine, pentylenediamine, hexamethylenediamine, heptylenediamine, octylenediamine, 1,4-cyclohexanediamine, diethanolamine, m-phenylenediamine, p-phenylenediamine, piperazine, 2-methylpiperazine, 2-methylimidazole, tetraethylenepentamine, triethylenetetramine, aniline, N-methylaniline, 4-methylaniline, diaminodiphenylmethane, 2,2-bis(4-aminophenyl)propane, diaminodiphenyl sulfone, diaminobenzophenone, cyanamide, melamine and dicyandiamide; optionally, the imine has a structure as shown in Formula (II): 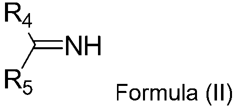
where R₄ and R₅ are independently selected from the group consisting of hydrogen, substituted or unsubstituted alkyl groups, and substituted or unsubstituted aryl groups; optionally, the organic nitrogen-containing compound is a primary amine compound.

9. The ammonium dihydrocarbylphosphinate according to any one of claims 1-8, wherein the ammonium dihydrocarbylphosphinate has any one of following structures: 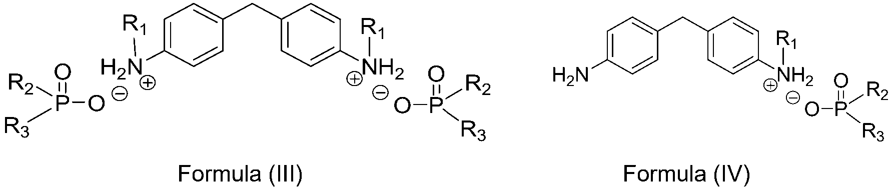 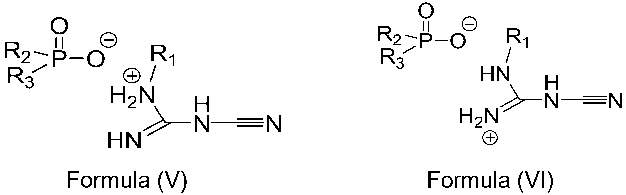 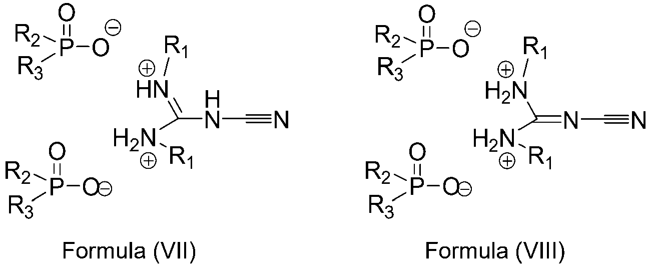 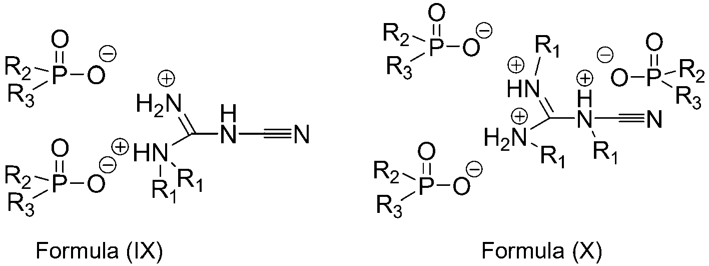 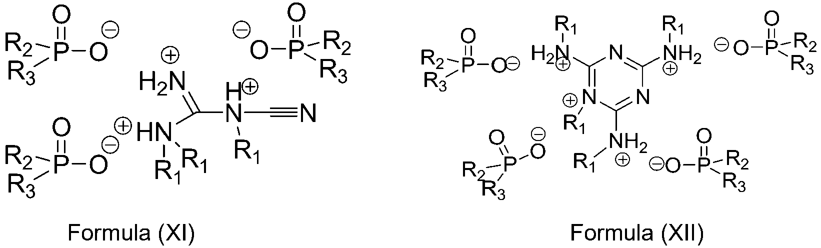 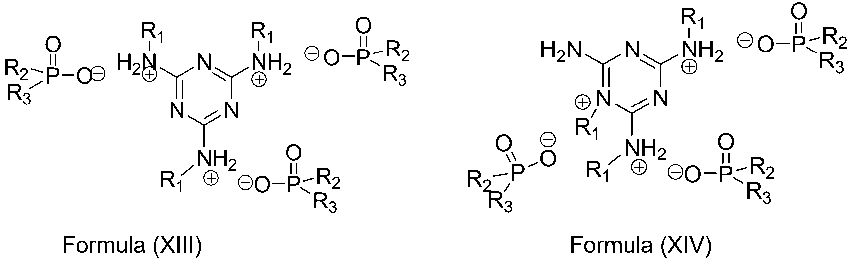 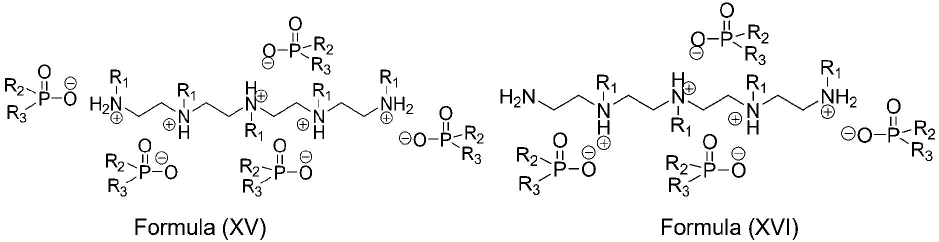 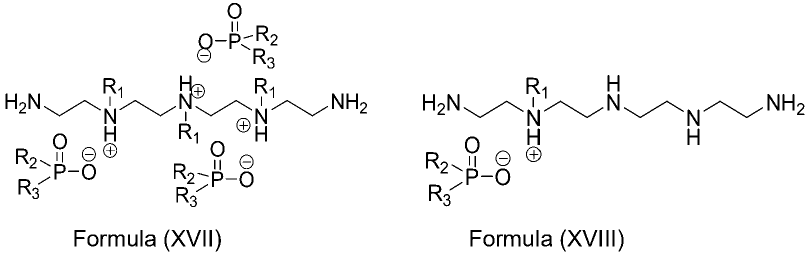 where R₁, R₂ and R₃ are as defined in claim 1.

10. A preparation method for preparing the ammonium dihydrocarbylphosphinate according to any one of claims 1-9, **characterized by** comprising following steps:
subjecting the alkyl dihydrocarbylphosphinate and nitrogen on at least one non-tertiary amine structure in NH₃ or the organic nitrogen-containing compound to the alkylation reaction to obtain the ammonium dihydrocarbylphosphinate.

11. The preparation method according to claim 10, wherein the alkyl dihydrocarbylphosphinate comprises any one or a combination of two selected from the group consisting of methyl dimethylphosphinate, methyl diethylphosphinate, methyl dipropylphosphinate, methyl ethylpropylphosphinate, methyl diphenylphosphinate, ethyl dimethylphosphinate, ethyl diethylphosphinate, ethyl dipropylphosphinate, ethyl ethylpropylphosphinate, propyl dimethylphosphinate, propyl diethylphosphinate, propyl dipropylphosphinate, propyl ethylpropylphosphinate, methyl methylphenylphosphinate and methyl diphenylphosphinate; optionally, the organic nitrogen-containing compound comprises any one or a combination of two selected from the group consisting of methylamine, ethylamine, propylamine, butylamine, pentylamine, hexylamine, heptylamine, octylamine, nonylamine, decylamine, dodecylamine, octadecylamine, cyclohexylamine, diethylamine, methylpropylamine, ethylenediamine, propylenediamine, butylenediamine, pentylenediamine, hexamethylenediamine, heptylenediamine, octylenediamine, 1,4-cyclohexanediamine, diethanolamine, m-phenylenediamine, p-phenylenediamine, piperazine, 2-methylpiperazine, 2-methylimidazole, tetraethylenepentamine, triethylenetetramine, aniline, N-methylaniline, 4-methylaniline, diaminodiphenylmethane, 2,2-bis(4-aminophenyl)propane, diaminodiphenyl sulfone, diaminobenzophenone, cyanamide, melamine and dicyandiamide.

12. The preparation method according to claim 10 or 11, wherein a molar ratio of total moles of nitrogen in the organic nitrogen-containing compound to the alkyl dihydrocarbylphosphinate is 10/1 - 1/10;
optionally, a temperature of the alkylation reaction is 0 °C - 250 °C;
optionally, a duration of the alkylation reaction is 0.1 hours - 30 hours.

13. A flame-retardant polymer material, **characterized in that** the flame-retardant polymer material comprises a flame retardant and a polymer material, wherein the flame retardant comprises the ammonium dihydrocarbylphosphinate according to any one of claims 1-9.

14. The flame-retardant polymer material according to claim 13, wherein the flame-retardant polymer material comprises 0.1 wt%-50 wt% of the flame retardant and 50 wt%-99.9 wt% of the polymer material;
optionally, the flame-retardant polymer material further comprises a functional additive; optionally, the functional additive is at least one selected from the group consisting of reinforcing agents, anti-dripping agents, stabilizers, pigments, dyes, char-forming catalysts, dispersants, nucleating agents, inorganic fillers, organic fillers and antioxidants;
optionally, the reinforcing agent is selected from glass fibers;
optionally, the anti-dripping agent is selected from Teflon;
optionally, the inorganic filler is at least one selected from the group consisting of silicon dioxide, mica, calcium carbonate, aluminum oxide, aluminum nitride, talc, calcium oxide, calcium silicate, glass fiber powder and silica;
optionally, the organic filler is at least one selected from the group consisting of polytetrafluoroethylene powder, polyphenylene sulfide powder and polyethersulfone powder;
optionally, a mass content of the functional additive in the flame-retardant polymer material is 5%-40%;
optionally, the flame-retardant polymer material further comprises a flame retardant Q; optionally, the flame retardant Q is at least one selected from the group consisting of phosphorus-based flame retardants, nitrogen-based flame retardants and boron-based flame retardants;
optionally, the phosphorus-based flame retardant is at least one selected from the group consisting of diphosphates, phosphazenes, dihydrocarbylphosphinates, 9,10-dihydro-9-oxa-10-phosphaphenanthrene-10-oxide derivatives, 6,6'-(1,2-ethanediyl)bis-6,6'-dioxide, HTP-6123, and phosphine oxide compounds;
optionally, the diphosphate is at least one selected from the group consisting of bisphenol A bis(diphenyl phosphate), resorcinol bis(diphenyl phosphate) and 1,3-phenylene tetrakis(2,6-dimethylphenyl) phosphate;
optionally, the phosphazene-based flame retardant is at least one selected from the group consisting of hexaphenoxycyclotriphosphazene, cyanophenoxyphosphazene, allylcyclophosphazene and cyclic biphenoxyphosphazene;
optionally, the dihydrocarbylphosphinate is at least one selected from the group consisting of aluminum diethylphosphinate, aluminum diisobutylphosphinate, aluminum dipropylphosphinate, and zinc diethylphosphinate;
optionally, the 9,10-dihydro-9-oxa-10-phosphaphenanthrene-10-oxide derivative is at least one selected from the group consisting of 6H-dibenzo[c,e][1,2]oxaphosphinin-6,6'-(1,4-ethanediyl)bis-6,6'-dioxide, and 6H-dibenzo[c,e][1,2]oxaphosphinin-6,6'-(1,4-phenylethanediyl)bis-6,6'-dioxide;
optionally, the phosphine oxide compound is p-xylylene bis(diphenylphosphine oxide);
optionally, the nitrogen-based flame retardant is at least one selected from the group consisting of melamine cyanurate, melamine polyphosphate and ammonium polyphosphate; optionally, the boron-based flame retardant is selected from zinc borate;
optionally, a mass content of the flame retardant Q in the flame-retardant polymer material is 0.5%-20%;
optionally, the polymer material is selected from the group consisting of thermoplastic polymer resins and thermosetting resins;
optionally, the thermoplastic polymer resin is at least one selected from the group consisting of polybutadiene, polyamide, polyester, polyphenylene ether, acrylonitrile-butadiene-styrene copolymer, polyethylene, polypropylene, polyvinyl chloride, polystyrene, polyimide, polyphenylene sulfide and polyether ether ketone;
optionally, the thermosetting resin is at least one selected from the group consisting of vinyl polyphenylene oxide resin, epoxy resin, cyanate ester resin, BT resin, bismaleimide resin, phenolic resin, polyurethane resin, thermosetting polyimide, unsaturated polyester resin, vinyl resin, benzoxazine resin, arylacetylene resin, and furan resin;
optionally, the thermosetting resin is at least one selected from the group consisting of vinyl polyphenylene oxide resin, epoxy resin, cyanate ester resin, BT resin, bismaleimide resin, and thermosetting polyimide;
optionally, the thermosetting resin is epoxy resin;
optionally, the epoxy resin is at least one selected from the group consisting of bisphenol A epoxy resin, bisphenol F epoxy resin, dicyclopentadiene epoxy resin, biphenyl epoxy resin, and naphthol epoxy resin.

15. The flame-retardant polymer material according to claim 13 or 14, wherein the flame-retardant polymer material is a flame-retardant epoxy resin material, wherein the flame-retardant epoxy resin material comprises a flame retardant and an epoxy resin, and the flame retardant comprises the ammonium dihydrocarbylphosphinate according to any one of claims 1-9;
optionally, the flame-retardant epoxy resin material comprises 0.1 wt%-18 wt% of the ammonium dihydrocarbylphosphinate and 82 wt%-99.9 wt% of the epoxy resin.
